# EUROPEAN PATENT SPECIFICATION

(11) **EP 1 842 844 B1**
(45) Date of publication and mention of the grant of the patent: **26.07.2017**
(21) Application number: 06005589.4
(22) Date of filing: 18.03.2006
(51) Int. Cl.: C07C 231/02

(54) **Continuous process for the production of monocarboxylic acid alkyl amides**
Kontinuierliches Verfahren zur Herstellung von Monocarbonsäure Alkylamiden
Procédé continu pour le production d'alkyl-amide d'acides monocarboxylique

(43) Date of publication of application: 10.10.2007
(73) Proprietor: Cognis IP Management GmbH, 40589 Dusseldorf (DE)
(72) Inventor: Bigorra Llosas, Joaquin Dr., 08201 Sabadell (ES); Raya, Javier, 08620, Sant Vicenc dels Horts (ES); Brau Balague, Emilio, 08950, Barcelona (ES); Seva, Manuel, 08755 Castellbisbal (Barcelona) (ES); Francia, Ivan, 08031, Barcelona (ES); Valls, Ramon, 08022, Barcelona (ES)
(74) Representative: BASF IP Association

(56) References cited:
- WO-A-94/15905
- US-B1- 6 723 877
- DATABASE WPI Section Ch, Week 200526 Derwent Publications Ltd., London, GB; Class E16, AN 2005-250164 XP002391046 -& RO 119 297 B1 (INST CERC PROD AUXILIARE ORGANICE) 30 July 2004 (2004-07-30)
- WENKER, HENRY: "The synthesis of D2-oxazolines and d2-thiazolidines from N-Acyl-2-aminoethanols" J. AM. CHEM. SOC., vol. 57, 1935, pages 1079-1080, XP002390393

## Description

### Object of the invention

The present invention relates to the area of green solvents and covers an improved process for the production of monocarboxylic acid alkyl amides as defined in the claims.

### State of the art

Carboxylic acid alkyl amides are well-known intermediates for the manufacture of polymers. Fatty acid alkanolamides, which form a sub-group of this species, are also applied as foam boosters in detergents, while particularly amides, which are based on dimethyl amine and medium chain fatty acids, are used as environmentally friendly, so-called "green" solvents, particularly in agriculture.

Usually, carboxylic acid alkyl amides are obtained from the reaction of triglycerides, carboxylic acids, their esters or halides with alkyl or alkanol amines (see, for example, US 5,388,644). Halides, particularly chlorides, however are difficult to handle, highly corrosive and toxic. In case of esters or triglycerides, alcohols or glycerol are obtained as an unwanted by-product which needs to be separated off and therefore makes the process expensive. The reaction between carboxylic acids and amines usually takes place in the gas phase, which makes it necessary to use closed reactors and high pressure. Due to salt formation, an excess of amine is necessary to achieve an acceptable conversion. Therefore, either non-reacted amine has to be removed after the amidation has been completed, which has a negative impact on the manufacturing costs, or a certain level of free amine in the product has to be accepted which is not always possible, especially in cases where the amides are used as environmentally friendly solvents. WO 94/15905 describes a method for amidation of carboxylic acids using a supported transition metal catalyst. RO 119 297 describes a method of obtaining N-substituted formamides, used as reaction media or intermediates in organic synthesis. US 6,723,877 B1 describes dimethylformamide synthesis via reactive distillation of methyl formate and dimethylamine. The synthesis of D2-oxazolines and d2-thiazolidines from N-Acyl-2-aminoethanols is disclosed in J. AM. Chem. Soc., vol 57, 1935, pages 1079-1080.

### Description of the invention

The present invention claims a continuous process for the production of monocarboxylic acid alkyl amides, which is characterised in that:
(a) fresh monocarboxylic acid is placed into a first reactor (R1) equipped with a distillation column (C1) and subjected to a reaction with gaseous alkyl amine to form a pre-mixture consisting of monocarboxylic acid alkyl amide and unreacted monocarboxylic acid, while the water of condensation is distilled off;
(b) said pre-mixture is transferred into a second reactor (R2) which is also equipped with a distillation column (C2) and subjected to a further reaction with gaseous alkyl amine to substantially convert all unreacted monocarboxylic acid into monocarboxylic acid alkyl amide; and
(c) all unreacted gaseous alkyl amine is transferred into the first reactor (R1) to start the amidation reaction;
wherein said monocarboxylic acids and said alkyl amines are reacted in stoichiometric amounts; characterised in that C₆-C₂₂ fatty acids are reacted with dimethyl amine and characterised in that the amidation is conducted at a pressure in the range of 1 to 5 bar.

According to the present invention it is possible for the first time to produce amides from various types of monocarboxylic acids and dimethyl amines
o continuously,
o in a closed process without generating unwanted by-products, and
o under stoichiometric and therefore rather economic conditions, which means that none of the compounds is used in excess.

### Monocarboxylic acids

A special advantage of the present invention is to produce amides from medium and long-chain C₆-C₂₂ fatty acids, such as caproic acid, caprylic acid, caprinic acid, lauric acid, myristic acid, palmitic acid, palmoleic acid, stearic acid, isostearic acid, oleic acid elaidic acid, linolic acid linoleic acid, ricinolic acid, 12-hydroxystearic acid, CLA, gadoleic acid, arachidonic acid, behenic acid, erucic acid and their technical mixtures, such as cocofatty acid, palmoil fatty acid, or tallow fatty acid.

In the present invention, C₆-C₂₂ fatty acids are reacted with dimethyl amine (DMA).

### Manufacturing process

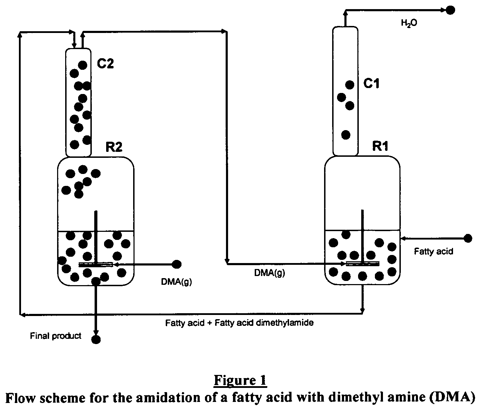

The process according to the present invention is illustrated in Figure 1, which shows a preferred example for the production of fatty acid dimethyl amides. A major advantage of the invention is that the reaction partners are reacted in stoichiometric amounts, which means that one mole of dimethyl amine is reacted with one mole of C₆-C₂₂ fatty acids . With the exception of condensation water, which is separated off from the reaction mixture in order to shift the balance to the product side, neither there are formed by-products, nor are measures necessary for re-cycling excessive amines or acids. Amidation takes place in two corresponding reactors which form a closed system. The reaction is typically conducted at a temperature in the range of 150 to 250, and preferably 200 to 220 °C, while the pressure ranges from 1 to 5, preferably 1.5 to 2 bar.

The first reactor serves for the production of a pre-mixture of monocarboxylic acids and their alkyl amides. For this purpose, fresh monocarboxylic acid as defined in the claims (or even their mixtures) are placed into the reactor (R1). Gaseous DMA - is introduced into the reactor, preferably by means of nozzles, or simply by bubbling through the liquid acid. The amidation takes place under vigorous stirring. The water of condensation is distilled off via column C1 and cooled outside of the reactor. Once the acid number has decreased to a value equivalent from 20 to 90, and preferably from 40 to 70 % b.w., the pre-mixture thus obtained leaves reactor R1 and enters the top of column C2 which is connected to reactor R2. Said reactor serves to complete the amidation. While the pre-mixture drops down through column C2, gaseous DMA is led in counter-current to react with the free fatty acid as defined in the claims in the pre-mixture. The monocarboxylic acid alkyl amides as defined in the claims are collected at the bottom of the reactor and may be recycled back to the bottom of column C2 to ensure that all fatty acid has been converted. Water formed during the condensation is distilled off via column C2. The product leaves the reactor until the acid value has reached the desired value, usually a number of less than 6. Any gaseous DMA which has not reacted with the free fatty acid in the pre-mixture is used to feed reactor R1 in order to close the circle. The monocarboxylic acid alkyl amide as defined in the claims thus obtained can be subjected to standard purification procedures, such as washing, deodorisation, etc.

In a preferred embodiment the gaseous alkyl amine is produced in situ from its aqueous solution. For this purpose said aqueous solution, e.g. an aqueous solution of DMA comprising about 60 % b.w. of water, is fed into the middle of a standard fractionation column. Typically, the conditions within the column are 120 to 140°C and 1.5 to 2.5 bar, while the temperature at the top of the column lies in the range of 50 to 60 °C. While the water is collected at the bottom, the gaseous DMA leaves the top of the column and is directly introduced into reactor R2.

## Claims

1. Continuous process for the production of monocarboxylic acid alkyl amides, **characterised in that**:
(a) fresh monocarboxylic acid is placed into a first reactor (R1) connected with a distillation column (C1) and subjected to a reaction with gaseous alkyl amine to form a pre-mixture consisting of mono carboxylic acid alkyl amide and unreacted monocarboxylic acid, while the water of condensation is distilled off;
(b) said pre-mixture is transferred into a second reactor (R2) also equipped with a distillation column (C2) and subjected to a further reaction with gaseous alkyl amine to substantially convert all unreacted monocarboxylic acid into monocarboxylic acid alkyl amide; and
(c) all unreacted gaseous alkyl amine is transferred into the first reactor (R1) to start the amidation reaction;
wherein said monocarboxylic acids and said alkyl amines are reacted in stoichiometric amounts; **characterised in that** C₆-C₂₂ fatty acids are reacted with dimethyl amine and **characterised in that** the amidation is conducted at a pressure in the range of 1 to 5 bar.

2. Process according to claim 1, **characterised in that** the amidation is conducted at a temperature in the range of 150 to 250 °C.

3. Process according to any of claims 1 or 2, **characterised in that** the gaseous alkyl amine is generated in situ by fractionation of the respective aqueous solution.

4. Process according to any of claims 1 to 3, **characterised in that** the pre-mixture leaving reactor R1 contains between 10 and 60 % b.w. monocarboxylic acid alkyl amide.

5. Process according to any of claims 1 to 4, **characterised in that** said pre-mixture and said gaseous alkyl amine are led in counter-current to form the alkyl amides in reactor R2.

6. Process according to any of claims 1 to 5, **characterised in that** said unreacted gaseous alkyl amine is conducted out from the reaction mixture in reactor R2 through column C2.

## Patentansprüche

1. Kontinuierliches Verfahren zur Herstellung von Monocarbonsäurealkylamiden, **dadurch gekennzeichnet, dass** man:
(a) frische Monocarbonsäure in einen ersten Reaktor (R1), der mit einer Destillationssäule (C1) verbunden ist, einbringt und einer Umsetzung mit gasförmigem Alkylamin zur Bildung einer Vormischung, die aus Monocarbonsäurealkylamid und nicht umgesetzter Monocarbonsäure besteht, unter Abdestillieren des Kondensationswassers unterwirft;
(b) die Vormischung in einen zweiten Reaktor (R2), die ebenfalls mit einer Destillationssäule (C2) ausgestattet ist, überführt und einer weiteren Umsetzung mit gasförmigem Alkylamin zur weitgehenden Umwandlung der gesamten nicht umgesetzten Monocarbonsäure in Monocarbonsäurealkylamid unterwirft und
(c) das gesamte nicht umgesetzte gasförmige Alkylamin zum Starten der Amidierungsreaktion in den ersten Reaktor (R1) überführt;
wobei die Monocarbonsäuren und die Alkylamine in stöchiometrischen Mengen umgesetzt werden; **dadurch gekennzeichnet, dass** C₆-₂₂-Fettsäuren mit Dimethylamin umgesetzt werden, und **dadurch gekennzeichnet, dass** die Amidierung bei einem Druck im Bereich von 1 bis 5 bar durchgeführt wird.

2. Verfahren nach Anspruch 1, **dadurch gekennzeichnet, dass** die Amidierung bei einer Temperatur im Bereich von 150 bis 250 °C durchgeführt wird.

3. Verfahren nach Anspruch 1 oder 2, **dadurch gekennzeichnet, dass** das gasförmige Alkylamin in situ durch Fraktionierung der entsprechenden wässrigen Lösung erzeugt wird.

4. Verfahren nach einem der Ansprüche 1 bis 3, **dadurch gekennzeichnet, dass** die aus dem Reaktor R1 austretende Vormischung zwischen 10 und 60 Gew.-% Monocarbonsäurealkylamid enthält.

5. Verfahren nach einem der Ansprüche 1 bis 4, **dadurch gekennzeichnet, dass** die Vormischung und das gasförmige Alkylamin zur Bildung der Alkylamide in Reaktor R2 im Gegenstrom geführt werden.

6. Verfahren nach einem der Ansprüche 1 bis 5, **dadurch gekennzeichnet, dass** nicht umgesetztes gasförmiges Alkylamin aus der Reaktionsmischung in Reaktor R2 durch Säule C2 ausgeleitet wird.

## Revendications

1. Procédé continu pour la production d'alkylamides d'acide monocarboxylique, **caractérisé en ce que** :
(a) un acide monocarboxylique frais est placé dans un premier réacteur (R1) raccordé à une colonne de distillation (C1) et soumis à une réaction avec une alkylamine gazeuse pour former un prémélange constitué d'alkylamide d'acide monocarboxylique et d'acide monocarboxylique n'ayant pas réagi, tandis que l'eau de condensation est séparée par distillation ;
(b) ledit prémélange est transféré dans un deuxième réacteur (R2) également équipé d'une colonne de distillation (C2) et soumis à une réaction supplémentaire avec une alkylamine gazeuse pour convertir sensiblement tout l'acide monocarboxylique n'ayant pas réagi en alkylamide d'acide monocarboxylique ; et
(c) la totalité de l'alkylamine gazeuse n'ayant pas réagi est transférée dans le premier réacteur (R1) pour démarrer la réaction d'amidation ;
dans lequel lesdits acides monocarboxyliques et lesdites alkylamines réagissent dans des quantités stoechiométriques ; **caractérisé en ce que** des acides gras en C₆-C₂₂ réagissent avec la diméthylamine et **caractérisé en ce que** l'amidation est conduite à une pression dans la plage de 1 à 5 bar.

2. Procédé selon la revendication 1, **caractérisé en ce que** l'amidation est conduite à une température dans la plage de 150 à 250 °C.

3. Procédé selon l'une quelconque des revendications 1 ou 2, **caractérisé en ce que** l'alkylamine gazeuse est générée *in situ* par fractionnement de la solution aqueuse respective.

4. Procédé selon l'une quelconque des revendications 1 à 3, **caractérisé en ce que** le prémélange quittant le réacteur R1 contient entre 10 et 60 % en poids d'alkylamide d'acide monocarboxylique.

5. Procédé selon l'une quelconque des revendications 1 à 4, **caractérisé en ce que** ledit prémélange et ladite alkylamine gazeuse sont conduits à contre-courant pour former les alkylamides dans le réacteur R2.

6. Procédé selon l'une quelconque des revendications 1 à 5, **caractérisé en ce que** ladite alkylamine gazeuse n'ayant pas réagi est conduite hors du mélange de réaction dans le réacteur R2 par l'intermédiaire de la colonne C2.
